# EUROPEAN PATENT SPECIFICATION

(11) **EP 1 948 104 B1**
(45) Date of publication and mention of the grant of the patent: **03.04.2013**
(21) Application number: 06806233.0
(22) Date of filing: 12.10.2006
(51) Int. Cl.: A61F 9/008

(54) **SYSTEM FOR CORRECTION OF OPHTHALMIC REFRACTIVE ERRORS**
SYSTEM ZUR KORREKTUR VON REFRAKTIONSFEHLERN DES AUGES
SYSTÈME POUR CORRIGER DES ERREURS DE RÉFRACTION OPHTALMIQUE

(30) Priority: 08.11.2005 DE 102005053297
(43) Date of publication of application: 30.07.2008
(73) Proprietor: Technolas Perfect Vision GmbH, 80992 München (DE)
(72) Inventor: MORITZ, Friedrich, 81541 München (DE); YOUSSEFI, Gerhard, 84028 Landshut (DE)
(74) Representative: Vossius & Partner
(86) International application number: PCT/EP2006/009878
(87) International publication number: WO 2007/054176

(56) References cited:
- EP-A2- 1 396 244
- WO-A-01/28410
- WO-A1-2004/052253
- US-A1- 2002 082 629

## Description

### Technical Field

The invention generally relates to a system and a method for correction of ophthalmic refractive errors and, more particularly, to a system and method for calculating a course of refractive treatment for correcting a refractive error.

### Background Art

Several techniques for correcting the vision of the eye have been proposed. The radial keratotomy technique provides slits in the cornea which allow the cornea to relax and reshape. The present techniques include photorefractive keratectomy ("PRK"), anterior lamellar keratectomy ("ALK"), laser in situ keratomileuses ("LASIK"), and thermal techniques such as laser thermal keratoplasty ("LTK"). All of these techniques strive to provide a relatively quick but lasting correction of vision.

WO 01/28477 A1 relates to a method and apparatus for multi-step correction of ophthalmic refractive errors. In a first step, gross decentrations of the refractive error are corrected, allowing the subsequent steps to be relatively symmetric in their treatment profile. After each step, the eye's refractive error is again measured, and the subsequent treatment is applied for the remaining error. With this known method, any biodynamic response which is observed after an initial step of treatment is taken into account for calculating the necessary treatment profile for correcting a residual refractive error.

EP 1396244 relates to a method and apparatus for multi-skp correction of prestyopia.

US 6,607,521 B2 relates to an apparatus for corneal surgery to correct a refractive error by ablating corneal tissue with a laser beam.

According to this known method, the step of hyperopic astigmatic correction and the step of myopic astigmatic correction are performed in combination to correct astigmatism. Thereafter, a step of spherical correction may be effected. These three steps may be followed by a fourth step for smoothing the laser irradiated surfaces. This known method shall eliminate the need to obtain a hyperopic shift rate upon astigmatic correction as an empirical value and over correction or under correction in certain portions of the ablation zone.

The object underlying the present invention is to provide a system and a method for calculating a course of refractive treatment for correcting a refractive error of a patient's eye.

This object is solved and the invention is defined with the features of the claims.

The present invention is based on the concept to combine at least a first treatment (in the following the main treatment) which provides an overcorrection of the intended correction with at least a second treatment (in the following a compensating treatment) which corrects said overcorrection. More specifically, according to a preferred embodiment of the invention, a myopic ablation pattern is combined with a hyperopic ablation pattern. The present invention has the advantage that post-operative spherical aberrations after a refractive laser treatment are controlled to a specific predetermined value, preferably minimum value. Known systems and methods providing a myopic ablation pattern often induce a negative spherical aberration. On the other hand, hyperopic treatment data show the opposite effect, i.e. they induce a positive spherical aberration. This change of the post-operative status of the eye having an increased spherical aberration can cause vision problems especially under conditions which cause the pupil to dilate, for example under dim light conditions. The patient's ability to see under such conditions can be severely limited. For example, a patient may not be able to drive a car by night. According to the present invention, this observed change in spherical aberration can be substantially reduced. Compared to other methods correcting for spherical aberration using wavefront measurement or topographic guided ablations, no additional information of the individual subject is necessary. A combination of both myopic and hyperopic ablation patterns can be used to adjust the post-op spherical aberration. For example, for a patient haying a refraction of -4 dioptres, the following treatment may be provided. Based on this refractive diagnostic eye data, a computer system calculates a first treatment profile which provides a slight overcorrection. As an example, this treatment profile will correspond to a treatment for correcting -5 dioptres. The resulting overcorrection will be compensated for by a second treatment profile, in this case by a hyperopic treatment of +1 dioptres. Preferably, the hyperopic treatment will take place immediately after the end of the myopic ablation treatment.

Alternatively, the order of the first and second treatment can be changed. Thus, for the above example, a hyperopic treatment of +1 dioptres may by followed by a myopic treatment of -5 dioptres.

As a further alternative the first or main treatment may be divided in at least two main treatment sub-profiles. The second or compensating treatment may be divided in at least two compensating treatment sub-profiles. The main treatment may for example comprise sub-profiles m1, m2 ...mx and the compensating treatment may comprise sub-profiles c1, c2, ...cy. The treatment may be performed with an order of the sub-profiles as follows: m1, c1, m2, c2, ...mx-1, cy, mx.

As another alternative the individual shots necessary for performing the main and the compensating profile are combined in one single shot file. Thus the over correction and under correction is performed as one unitary treatment.

The effect of the present invention on post-op spherical aberration can be adjusted or optimized by selecting the amount of initial overcorrection and the corresponding amount of secondary correction as well as the corresponding optical zone sizes.

According to the invention, the shot file for the first treatment is calculated with reference to a corresponding first optical zone and the shot file for the second treatment is calculated with reference to a corresponding second optical zone. Preferably, the size of the first optical zone is different from the size of the second optical zone. Most preferably, the size of the optical zone for performing a hyperopic treatment is smaller than the size of the optical zone for the myopic treatment.

The system and the method according to the present invention can be used for hyperopic and myopic corrections with or without a cylinder.

The present invention will be further described by way of the following examples and the drawings, in which:
Figure 1 schematically shows a cross section of a patient's eye and
Figure 2 shows an example of a system for performing techniques according to the invention.

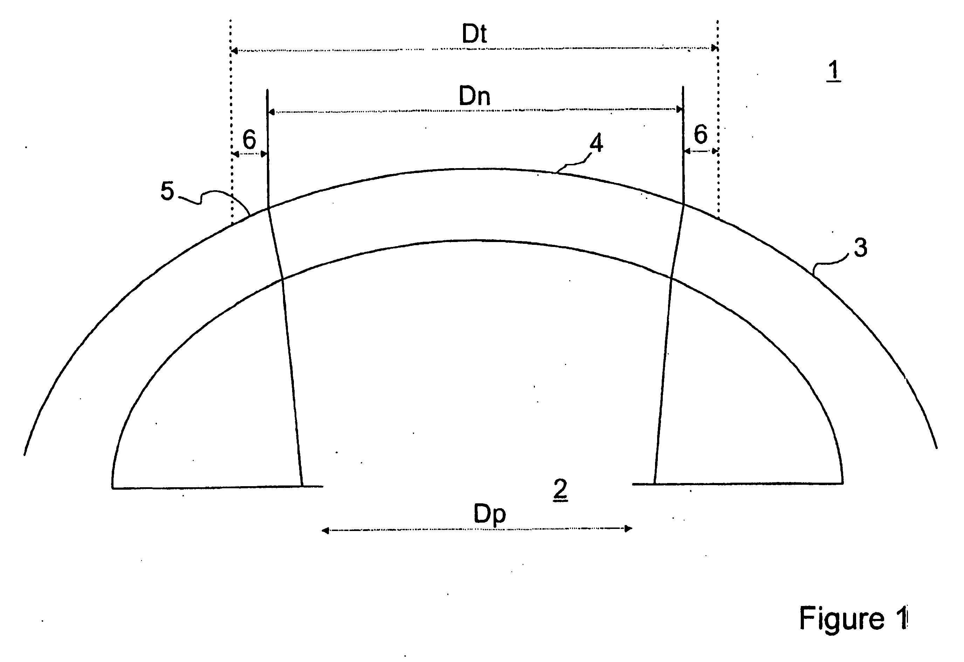

Figure 1 shows a cross-section of a patient's eye 1, having a pupil 2 and a cornea 3. As shown in Figure 1, the diameter Dn of a nominal optical zone 4 is selected to be greater than the diameter Dp of the pupil 2 of the patient's eye 1 under dim light condition. More precisely, the diameter Dn of a usually circular nominal optical zone 4 is at least 0.2 to 0.5 mm greater than the diameter Dp of the pupil 2 of the patient's eye 1 under dim light condition. During laser treatment, the cornea 3 is treated within a treatment zone 5 having a diameter Dt wherein the treatment zone comprises the optical zone 4 and an annular-like transition zone 6 surrounding the optical zone. Thus, the optically full corrected zone will be greater than the pupil size of a patient under dim light condition or a typical size of a patient when driving a car at night.

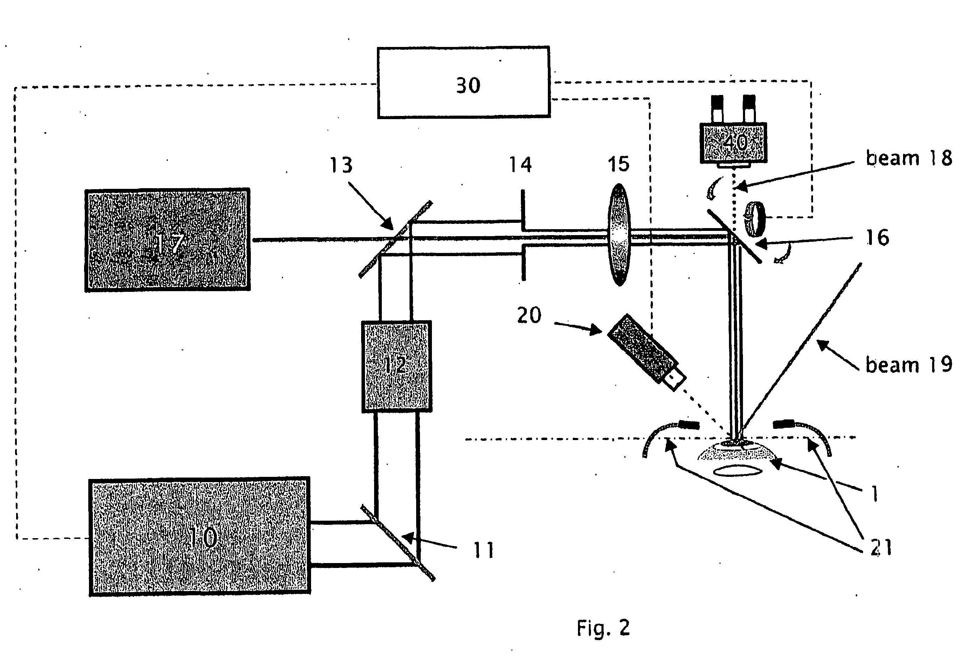

Figure 2 schematically shows an example of the system for performing the techniques according to the present invention. It comprises an excimer laser 10, which outputs a laser beam which by means of an optical system is directed to a patient's eye 1. The optical system in this example comprises a first deviation mirror 11, a beam homogenising system 12, a second deviation mirror 13, an aperture 14, a lens 15 and a scanning mirror 16. The system further comprises an aiming beam laser diode 17 which outputs an aiming beam through the second deviation mirror 13 on the same optical path as the excimer laser beam to the patient's eye 1. The system additionally comprises a fixation laser which provides a preferably red flashing fixation laser beam 18 through the scanning mirror to the patient's eye 1. The system further comprises a preferably green focussing laser beam 19 which is directed to the patient's eye 1 at an angle with respect to the optical path of the excimer laser beam. In this system, an eye tracker 20 with a corresponding infrared illumination system 21 is provided. A computer system 30 is connected to the excimer laser 10, the scanning mirror 16 and the eye tracker 20 via data communication lines (see dashed lines). Figure 2 also schematically indicates an operation microscope 40 which with respect to the patient's eye is arranged behind the scanning mirror 16. The treatment zone is schematically indicated by a dotted dashed line.

A system for providing a course of refractive treatment for correcting a refractive error, in particular a spherical error generally comprises a computer system that receives refractive eye data from a refractive tool. This refractive tool may be a phoropter (not shown) for determining the refractive properties of a patient's eye. The computer system calculates at least the first and second treatment profile which is used in combination with a refractive surgical correction system for correcting refractive errors. Such a refractive surgical correction system is preferably an excimer laser eye surgery system which is used for ablating corneal tissue with a laser beam emitted from a laser source and delivered onto a cornea of a patient's eye with a light delivering optical system. The computer system 30 is generally a personal computer compatible with the IBM PC by International Business Machines, preferably including a high-powered processor. The laser system 10 can be a variety of systems, including the Keracor 217 by Technolas GmbH of Dornach, Germany. Generally, the computer system 30 runs the software which develops a course of treatment based on parameters provided by the physician as well as refractive data. It can employ a variety of algorithms, generally depending on the type of excimer laser system 10. The excimer laser system 10 preferably employs a relatively large fixed spot size, for example, algorithms described in WO 96/11655 can be used to develop a course of treatment based on a first treatment profile for overcorrection and a second treatment profile for correcting the overcorrection.

The refractive diagnostic eye data may be described as shown in the following:
S/C/A
wherein S denotes the sphere in dioptres, C denotes the cylinder in dioptres and A denotes the axis of the astigmatism. Herein, the minus cylinder convention is used. More specifically, the S/C/A represent the respective input values for calculating the treatment profile for correction of a refractive error of a patient's eye.

The system according to the present invention provides a course of refractive treatment which comprises a computer system that receives refractive eye data indicative of a refractive error, preferably a spherical error of the eye. The computer system calculates at least a first treatment profile for performing a main treatment which, however, provides an overcorrection of the intended correction. For example, the intended correction is defined as S/0/0 whereas the first treatment profile provides an overcorrection of S+F1·S/0/0. The value F1 is a constant in the range of 0.05 to 0.3, preferably in the range of 0.05 to 0.15. When calculating this first treatment profile, a first optical zone having a diameter D1 is taken into account. The computer system further calculates at least a second treatment profile suitable to correct said overcorrection which can be described in the present case as -F1·S/0/0. This second treatment profile is calculated with reference to a second optical zone OZ2 having a diameter D2 which is smaller than diameter D1 of the first optical zone OZ1.

Thus, the following two steps would be combined.

| | |
|---|---|
| 1) S+F1·S/0/0 | OZ1=OZnominal |
| 2) -F1·S/0/0 | OZ2<OZ1 |

According to a embodiment of the present invention, the computer system comprises a first treatment profile which represents the intended correction which may be described as S/0/0. The computer system further calculates a second treatment profile suitable to correct an overcorrection, i.e. -F1·S/0/0 and further calculates a third treatment profile providing said overcorrection, i.e. F1·S/0/0. The first treatment profile is calculated with reference to the first optical zone OZ1, the second treatment profile is calculated with reference to a second optical zone OZ2 and the third treatment profile is calculated with reference to a third optical zone OZ3. Herein, the diameter D1 of the first optical zone OZ1 is greater than the diameter of the second and the third optical zones OZ2 and OZ3. Preferably, the diameter D2 of the second optical zone OZ2 is greater than the diameter D3 of the third optical zone OZ3. For this embodiment, the following three steps are calculated.

| | | |
|---|---|---|
| 1) S/0/0 | OZ1=OZ | |
| 2) -F1·S/0/0 | OZ2<OZ1 | |
| 3) F1·S/0/0 | OZ3≤OZ2 | F1= 0,05 ...0.3 |

### Example

In this example, the intended treatment for correction of a spherical error of a patient is based on the following data:

| | |
|---|---|
| Refraction -6/0/0 | optical zone = 7mm |

This intended treatment may be divided into the following steps.

| | |
|---|---|
| 1) -6/0/0 | optical zone = 7mm |
| 2) +0.5/0/0 | optical zone = 6mm |
| 3) -0.5/0/0 | optical zone = 5mm |

In this example, F1 =0.083.

A treatment is calculated with reference to a first, second and third optical zone. The first optical zone OZ1 corresponds to said nominal optical zone.

With reference to the diameter D1 of said first optical zone a diameter D2 of the second optical zone OZ2 is selected from a range of D1-0.5 mm to D1-1.5 mm. With reference to the diameter D1 of said first optical zone the diameter D3 of the third optical zone OZ3 is selected from a range of D1 to D1-2.5 mm. The selection of the respective size of the optical zones has the advantage that viewing ability under dim light condition is improved.

The foregoing disclosure and description of the embodiments are illustrative and explanatory thereof, and various changes in the illustrated construction and method of operation may be made without departing from the scope of the invention.

## Claims

1. A system for providing a course of refractive treatment for correcting a refractive error comprising:
a computer system (30) adapted to receive refractive eye data indicative of a refractive error of the eye, adapted to calculate at least a first treatment profile, which based upon the refractive eye data provides an overcorrection and adapted to calculate at least a second treatment profile suitable to correct said overcorrection, **characterized in that** the refractive error is S/C/A, wherein S denotes myopic or hyperopic sphere in dioptres C denotes cylinder in dioptres and A denotes axis of astigmatism, the first treatment profile provides a correction of S+ F1*S/0/0, F1 is in the range of 0.05 to 0.3

2. The system of claim 1, wherein F1 is in the range of 0.05 to 0.15.

3. The system of any of claims 1 or 2, wherein the first treatment profile is provided for overcorrection of a myopic error resulting in a hyperopic error and wherein the second treatment profile is provided for correcting the resulting hyperopic error.

4. The system of any of claims 1 or 2, wherein the first treatment profile is provided for overcorrection of a hyperopic error resulting in a myopic error and wherein the second treatment profile is provided for correcting the resulting myopic error.

5. The system of any of claims 1 to 4, wherein any of the at least one first and/or second treatment profile is divided in at least two treatment sub-profiles with corresponding optical zones.

6. The system of any of claims 1 to 5, wherein the computer system (30) calculates the first treatment profile for a corresponding first optical zone having a first size and calculates the second treatment profile for a corresponding second optical zone having a second size, wherein the second size is smaller than the first size.

7. The system of any of claims 1 to 5, wherein the computer system (30) calculates a single shot file comprising the first treatment profile for a corresponding first optical zone having a first size and the second treatment profile for a corresponding second optical zone having a second size, wherein the second size is smaller than the first size.

8. The system of any of claims 1 to 7, further comprising
a refractive tool providing said refractive eye data,
a refractive surgical correction system (10) for correcting refractive error,
said computing system (30) being adapted to receive the refractive eye data from the refractive tool and being adapted to provide control data to the refractive surgical correction system (10), wherein said control data correspond to the at least one first treatment profile and the at least one second treatment profile.

9. The system of claim 8, wherein the at least one first and second treatment profile is calculated for a predetermined excimer laser eye surgery system (10).

10. A method of providing a course of refractive treatment for correcting a refractive error comprising:
receiving refractive eye data indicative of a refractive error of the eye, calculating at least a first treatment profile, which based upon the refractive eye data provides an overcorrection and calculating at least a second treatment profile suitable to correct said overcorrection, **characterized in that** the refractive error is S/C/A, wherein S denotes myopic or hyperopic sphere, in dioptres C denotes cylinder in dioptres and A denotes axis of astigmatism, the first treatment profile provides a correction of S+ F1*/S/C/A, and the second treatment profile provides a correction of -F1*S/0/0, wherein F1 is in the range of 0.05 to 0.3.

11. The method of claim 10, wherein F1 is in the range of 0.05 to 0.15.

12. The method of any of claims 10 or 11, wherein the first treatment profile is provided for overcorrection of a myopic error resulting in a hyperopic error and wherein the second treatment profile is provided for correcting the resulting hyperopic error.

13. The method of any of claims 10 to 12, wherein the first treatment profile is provided for overcorrection of a hyperopic error resulting in a myopic error and wherein the second treatment profile is provided for correcting the resulting myopic error.

14. The method of any of claims 10 to 13, wherein any of the at least one first and/or second treatment profile is divided in at least two treatment sub-profiles with corresponding optical zones.

15. The method of any of claims 10 to 14, wherein the step of calculating the first treatment profile is performed for a corresponding first optical zone having a first size and the step of calculating the second treatment profile is performed for a corresponding second optical zone having a second size, wherein the second size is smaller than the first size.

16. The method of any of claims 10 to 15, wherein a single shot file is determined for the first treatment profile based on a corresponding first optical zone having a first size and for the second treatment profile based on a corresponding second optical zone having a second size, wherein the second size is smaller than the first size.

## Patentansprüche

1. System zur Bereitstellung eines Verlaufs einer refraktiven Behandlung zur Korrektur eines Brechungsfehlers, das aufweist:
ein Computersystem (30), das dafür geeignet ist, refraktive Augendaten, die für einen Brechungsfehler des Auges kennzeichnend sind, zu empfangen, das dafür geeignet ist, mindestens ein erstes Behandlungsprofil, das beruhend auf den refraktiven Augendaten eine Überkorrektur liefert, zu berechnen, und das dafür geeignet ist, mindestens ein zweites Behandlungsprofil, das geeignet ist, die Überkorrektur zu korrigieren, zu berechnen, **dadurch gekennzeichnet, dass** der Brechungsfehler aus S/C/A besteht, wobei S eine myope oder hypermetropische Sphäre in Dioptrien bezeichnet, C einen Zylinder in Dioptrien bezeichnet und A eine Astigmatismusachse bezeichnet, das erste Behandlungsprofil eine Korrektur von S+ F1*S/C/A bereitstellt und das zweite Behandlungsprofil eine Korrektur von -F1*S/0/0 bereitstellt, wobei F1 im Bereich von 0,05 bis 0,3 liegt.

2. System nach Anspruch 1, wobei F 1 im Bereich von 0,05 bis 0,15 liegt.

3. System nach einem der Ansprüche 1 oder 2, wobei das erste Behandlungsprofil zur Überkorrektur eines myopen Fehlers vorgesehen ist, wobei es zu einem hypermetropischen Fehler führt, und wobei das zweite Behandlungsprofil zur Korrektur des resultierenden hypermetropischen Fehlers vorgesehen ist.

4. System nach einem der Ansprüche 1 oder 2, wobei das erste Behandlungsprofil zur Überkorrektur eines hypermetropischen Fehlers vorgesehen ist, wobei es zu einem myopen Fehler führt, und wobei das zweite Behandlungsprofil zur Korrektur des resultierenden myopen Fehlers vorgesehen ist.

5. System nach einem der Ansprüche 1 bis 4, wobei irgendeines des mindestens einen ersten und/oder zweiten Behandlungsprofils in mindestens zwei Behandlungsteilprofile mit entsprechenden optischen Zonen unterteilt ist.

6. System nach einem der Ansprüche 1 bis 5, wobei das Computersystem (30) das erste Behandlungsprofil für eine entsprechende erste optische Zone mit einer ersten Größe berechnet, und das zweite Behandlungsprofil für eine entsprechende zweite optische Zone mit einer zweiten Größe berechnet, wobei die zweite Größe kleiner als die erste Größe ist.

7. System nach einem der Ansprüche 1 bis 5, wobei das Computersystem (30) eine einzelne Schussdatei berechnet, die das erste Behandlungsprofil für eine entsprechende erste optische Zone mit einer ersten Größe und das zweite Behandlungsprofil für eine entsprechende zweite optische Zone mit einer zweiten Größe aufweist, wobei die zweite Größe kleiner als die erste Größe ist.

8. System nach einem der Ansprüche 1 bis 7, das ferner aufweist:
ein refraktives Instrument, das die refraktiven Augendaten bereitstellt,
ein refraktives chirurgisches Korrektursystem (10) zur Korrektur eines Brechungsfehlers,
wobei das Berechnungssystem (30) dafür geeignet ist, die refraktiven Augendaten vom refraktiven Instrument zu empfangen und Steuerdaten an das refraktive chirurgische Korrektursystem (10) zu liefern, wobei die Steuerdaten dem mindestens einen ersten Behandlungsprofil und dem mindestens einen zweiten Behandlungsprofil entsprechen.

9. System nach Anspruch 8, wobei das mindestens eine erste und zweite Behandlungsprofil für ein vorbestimmtes Excimerlaser-Augenchirurgiesystem (10) berechnet wird.

10. Verfahren zum Bereitstellen eines Verlaufs einer refraktiven Behandlung zur Korrektur eines Brechungsfehlers, das aufweist:
Empfangen von refraktiven Augendaten, die für einen Brechungsfehler des Auges kennzeichnend sind, Berechnen mindestens eines ersten Behandlungsprofils, das beruhend auf den refraktiven Augendaten eine Überkorrektur liefert und Berechnen mindestens eines zweiten Behandlungsprofils, das geeignet ist, die Überkorrektur zu korrigieren, **dadurch gekennzeichnet, dass** der Brechungsfehler aus S/C/A besteht,
wobei S eine myope oder hypermetropische Sphäre in Dioptrien bezeichnet, C einen Zylinder in Dioptrien bezeichnet und A eine Astigmatismusachse bezeichnet, das erste Behandlungsprofil eine Korrektur von S+ F1*S/C/A bereitstellt und das zweite Behandlungsprofil eine Korrektur von -F1*S/0/0 bereitstellt, wobei F1 im Bereich von 0,05 bis 0,3 liegt.

11. Verfahren nach Anspruch 10, wobei F 1 im Bereich von 0,05 bis 0,15 liegt.

12. Verfahren nach einem der Ansprüche 10 oder 11, wobei das erste Behandlungsprofil zur Überkorrektur eines myopen Fehlers vorgesehen ist, wobei es zu einem hypermetropischen Fehler führt, und wobei das zweite Behandlungsprofil zur Korrektur des resultierenden hypermetropischen Fehlers vorgesehen ist.

13. Verfahren nach einem der Ansprüche 10 bis 12, wobei das erste Behandlungsprofil zur Überkorrektur eines hypermetropischen Fehlers vorgesehen ist, wobei es zu einem myopen Fehler führt, und wobei das zweite Behandlungsprofil zur Korrektur des resultierenden myopen Fehlers vorgesehen ist.

14. Verfahren nach einem der Ansprüche 10 bis 13, wobei irgendeines des mindestens einen ersten und/oder zweiten Behandlungsprofils in mindestens zwei Behandlungsteilprofile mit entsprechenden optischen Zonen unterteilt wird.

15. Verfahren nach einem der Ansprüche 10 bis 14, wobei der Schritt des Berechnens des ersten Behandlungsprofils für eine entsprechende erste optische Zone durchgeführt wird, die eine erste Größe aufweist, und der Schritt des Berechnens des zweiten Behandlungsprofils für eine entsprechende zweite optische Zone durchgeführt wird, die eine zweite Größe aufweist, wobei die zweite Größe kleiner als die erste Größe ist.

16. Verfahren nach einem der Ansprüche 10 bis 15, wobei eine einzelne Schussdatei für das erste Behandlungsprofil, das auf einer entsprechenden ersten optischen Zone mit einer erste Größe beruht, und für das zweite Behandlungsprofil bestimmt wird, das auf einer entsprechenden zweiten optischen Zone mit einer zweiten Größe beruht, wobei die zweite Größe kleiner als die erste Größe ist.

## Revendications

1. Système pour appliquer un programme de traitement réfractif pour corriger une erreur de réfraction comprenant :
un système informatique (30) conçu pour recevoir des données de réfraction de l'oeil indicatives d'une erreur de réfraction de l'oeil, conçu pour calculer au moins un premier profil de traitement, qui, sur la base des données de réfraction de l'oeil, applique une surcorrection et conçu pour calculer au moins un deuxième profil de traitement approprié pour corriger ladite surcorrection, **caractérisé en ce que** l'erreur de réfraction est S/C/A, où S indique une sphère myopique ou hyperopique en dioptries, C indique un cylindre en dioptries et A indique l'axe d'astigmatisme, le premier profil de traitement applique une correction de S+ F1*S/C/A, et le deuxième profil de traitement applique une correction de -F1*S/0/0, où F1 est dans la plage de 0,05 à 0,3.

2. Système selon la revendication 1, dans lequel F1 est dans la plage de 0,05 à 0,15.

3. Système selon l'une quelconque des revendications 1 et 2, dans lequel le premier profil de traitement est appliqué pour la surcorrection d'une erreur myopique résultant en une erreur hyperopique, et dans lequel le deuxième profil de traitement est appliqué pour la correction de l'erreur hyperopique résultante.

4. Système selon l'une quelconque des revendications 1 et 2, dans lequel le premier profil de traitement est appliqué pour la surcorrection d'une erreur hyperopique résultant en une erreur myopique, et dans lequel le deuxième profil de traitement est appliqué pour la correction de l'erreur myopique résultante.

5. Système selon l'une quelconque des revendications 1 à 4, dans lequel l'un quelconque desdits au moins un premier et/ou deuxième profil de traitement est divisé en au moins deux sous-profils de traitement avec des zones optiques correspondantes.

6. Système selon l'une quelconque des revendications 1 à 5, dans lequel le système informatique (30) calcule le premier profil de traitement pour une première zone optique correspondante ayant une première taille et calcule le deuxième profil de traitement pour une deuxième zone optique correspondante ayant une deuxième taille, dans lequel la deuxième taille est inférieure à la première taille.

7. Système selon l'une quelconque des revendications 1 à 5, dans lequel le système informatique (30) calcule un fichier de tir unique comprenant le premier profil de traitement pour une première zone optique correspondante ayant une première taille et le deuxième profil de traitement pour une deuxième zone optique correspondante ayant une deuxième taille, dans lequel la deuxième taille est inférieure à la première taille.

8. Système selon l'une quelconque des revendications 1 à 7, comprenant en outre :
un outil réfractif fournissant lesdites données de réfraction de l'oeil,
un système de correction chirurgicale réfractive (10) pour corriger une erreur de réfraction,
ledit système informatique (30) étant conçu pour recevoir les données de réfraction de l'oeil de l'outil réfractif et étant conçu pour fournir des données de commande au système de correction chirurgicale réfractive (10), dans lequel lesdites données de commande correspondent au dit au moins un premier profil de traitement et au dit au moins un deuxième profil de traitement.

9. Système selon la revendication 8, dans lequel lesdits au moins un premier et deuxième profils de traitement sont calculés pour un système de chirurgie de l'oeil à laser excimère (10) prédéterminé.

10. Procédé d'application d'un programme de traitement réfractif pour corriger une erreur de réfraction comprenant :
la réception de données de réfraction de l'oeil indicatives d'une erreur de réfraction de l'oeil, le calcul d'au moins un premier profil de traitement, qui, sur la base des données de réfraction de l'oeil, applique une surcorrection et le calcul d'au moins un deuxième profil de traitement approprié pour corriger ladite surcorrection, **caractérisé en ce que** l'erreur de réfraction est S/C/A, où S indique une sphère myopique ou hyperopique en dioptries, C indique un cylindre en dioptries et A indique l'axe d'astigmatisme, le premier profil de traitement applique une correction de S+ F1*S/C/A, et le deuxième profil de traitement applique une correction de -F1*S/0/0, où F1 est dans la plage de 0,05 à 0,3.

11. Procédé selon la revendication 10, dans lequel F1 est dans la plage de 0,05 à 0,15.

12. Procédé selon l'une quelconque des revendications 10 et 11, dans lequel le premier profil de traitement est appliqué pour la surcorrection d'une erreur myopique résultant en une erreur hyperopique, et dans lequel le deuxième profil de traitement est appliqué pour corriger l'erreur hyperopique résultante.

13. Procédé selon l'une quelconque des revendications 10 à 12, dans lequel le premier profil de traitement est appliqué pour la surcorrection d'une erreur hyperopique résultant en une erreur myopique et dans lequel le deuxième profil de traitement est appliqué pour la correction de l'erreur myopique résultante.

14. Procédé selon l'une quelconque des revendications 10 à 13, dans lequel l'un quelconque desdits au moins un premier et/ou deuxième profils de traitement est divisé en au moins deux sous-profils de traitement avec des zones optiques correspondantes.

15. Procédé selon l'une quelconque des revendications 10 à 14, dans lequel l'étape de calcul du premier profil de traitement est effectuée pour une première zone optique correspondante ayant une première taille et l'étape de calcul du deuxième profil de traitement est effectuée pour une deuxième zone optique correspondante ayant une deuxième taille, dans lequel la deuxième taille est inférieure à la première taille.

16. Procédé selon l'une quelconque des revendications 10 à 15, dans lequel un fichier de tir unique est déterminé pour le premier profil de traitement sur la base d'une première zone optique correspondante ayant une première taille et pour le deuxième profil de traitement sur la base d'une deuxième zone optique correspondante ayant une deuxième taille, dans lequel la deuxième taille est inférieure à la première taille.
